# EUROPÄISCHE PATENTSCHRIFT

(11) **EP 4 161 449 B1**
(45) Veröffentlichungstag und Bekanntmachung des Hinweises auf die Patenterteilung: **09.07.2025**
(21) Anmeldenummer: 21728561.8
(22) Anmeldetag: 26.05.2021
(51) Int. Cl.: A61F 2/44, A61F 2/46

(54) **MONOLITHISCHER ZERVIKALER ODER LUMBALER IMPLANTATKÖRPER**
MONOLITHIC CERVICAL OR LUMBAR IMPLANT BODY
CORPS D'IMPLANT CERVICAL OU LOMBAIRE D'UN SEUL TENANT

(30) Priorität: 04.06.2020 DE 102020114862; 14.09.2020 DE 102020123848
(43) Veröffentlichungstag der Anmeldung: 12.04.2023
(73) Patentinhaber: NGMedical GmbH, 66620 Nonnweiler (DE)
(72) Erfinder: BACKES, Hermann, 66640 Namborn (DE); WEILAND, Nino, 66620 Nonnweiler (DE)
(74) Vertreter: Meissner Bolte Partnerschaft mbB
(86) Internationale Anmeldenummer: PCT/EP2021/064086
(87) Internationale Veröffentlichungsnummer: WO 2021/244922

(56) Entgegenhaltungen:
- EP-A1- 3 406 226
- CN-A- 107 349 034
- US-A1- 2018 256 336
- US-A1- 2019 117 410
- US-A1- 2019 343 652
- US-A1- 2019 350 673

## Beschreibung

Die Erfindung betrifft einen monolithischen zervikalen oder lumbalen Implantatkörper zur Fusion von Wirbelsäulensegmenten mit einer, voneinander unter Einhaltung eines Zwischenraumes beabstandeten Ober- und einer Unterseite, wobei die Ober- und die Unterseite durch Stege verbunden sind, weiterhin die Ober- und Unterseite mit Ausnahme von formgebenden und strukturverstärkenden Randbereichen als Fläche mit Durchbrüchen ausgebildet sind und einen Winkel einschließen, mindestens einer vollständig oder teilweise offenen Seitenfläche zum Einbringen von Knochenmaterial oder Knochenersatzmaterial und einer weiteren Seitenfläche mit Stabilisierungsbereich und einer Ausnehmung zur zeitweisen Aufnahme eines chirurgischen Instrumentes im Stabilisierungsbereich, gemäß Oberbegriff des Anspruches 1.

Aus der US 2019/000636 A1 ist ein Wirbelsäulenimplantat mit einer Einheitsstruktur vorbekannt, die unter Verwendung eines 3D-Druckers erzeugt wird.

Die US 2008/249627 A1 betrifft Zwischenwirbelimplantate mit Gewinde zum Einsatz in der menschlichen Wirbelsäule. Die vorbekannten Implantate weisen funktionelle Gewinde auf, die dem Fixieren im Wirbel dienen.

Aus der gattungsbildenden DE 10 2009 014 184 A1 ist ein Implantat zur Fusion von Wirbelsäulensegmenten vorbekannt, welches monolithisch aufgebaut ist. Mindestens Teile der Oberfläche des Implantates verfügen über eine strukturbildende Porosität. Das Volumen des Implantats weist eine hohe Dichte auf. Weiterhin umfasst das Volumen eine Anzahl von richtungsorientierten und/oder willkürlich angeordneten, in verschiedene Richtungen zeigende Durchgänge. Die Durchgänge sind von, die Festigkeit des Implantates erhöhenden, Stabilisierungsflächen umgeben, begrenzt oder unterbrochen.

Vorzugsweise sind die Durchgänge nach DE 10 2009 014 184 A1 als Wabenstruktur ausgebildet. Derartige sechseckige Hohlräume stellen ein gutes Verhältnis der Fläche des damit geschaffenen Durchganges in der Festigkeit der den Hohlraum begrenzenden Struktur dar.

Ausgehend von der größten Oberflächenseite des monolithischen Implantates wie vorbekannt, verlaufen die Durchgänge in senkrechter Richtung. Bei einer bevorzugten Ausbildung werden die Durchgänge in ihrem richtungsorientierten Verlauf von mindestens einem Freiraum unterbrochen.

In einer weiteren Ausführungsform verlaufen die Durchgänge in einer oder mehreren, von der Senkrechten abweichenden Richtung, wodurch sich das Einsinkverhalten verbessert.

In einer Weiterbildung weist das monolithische Implantat ein leicht angedeutetes keilförmiges Profil auf, um zum einen den Implantationsvorgang zu erleichtern und zum anderen der Form der Wirbelsäule in anatomischer Hinsicht zu entsprechen.

Das vorbekannte Implantat besitzt eine Bohrung zur zeitweiligen Aufnahme von chirurgischen Instrumenten, um das Setzen des Implantates zu erleichtern.

Außerdem ist mindestens eine Öffnung im Implantat vorhanden, welche zur Applikation von Knochenersatzmaterial dient. Das vorbekannte Implantat kann im Zuge eines Sinterverfahrens und/oder mittels Elektronenstrahlschmelzverfahren hergestellt werden.

Grundsätzlich bekannt sind weiterhin additive Verfahren zur Herstellung von Implantaten. Hierbei kommt auch das sogenannte selektive Laserschmelzen zum Einsatz.

Bei einem solchen Verfahren wird der zu verarbeitende Werkstoff pulverförmig in einer dünnen Schicht auf einer Grundplatte aufgebracht.

Dieser Werkstoff wird dann mittels Laserstrahlen lokal vollständig umgeschmolzen und bildet nach der Erstattung eine feste Materialschicht. Auf diese Weise lassen sich individuelle dreidimensionale Körper auch mit Hinterschnitten und Hohlräumen realisieren.

Werden bei mittels additiver Verfahren hergestellten Implantaten Gewindebohrungen nötig, um zum Beispiel chirurgische Instrumente zu fixieren, ist es bisher notwendig, die Gewinde entweder grundsätzlich spangebend, das heißt schneidend, auszubilden oder additiv erzeugte Gewinde nachzuschneiden oder nachzufurchen, um die entsprechende Präzision sicherzustellen. Ein Gewindeschneiden oder ein Gewindenachschneiden erfordert jedoch den Einsatz von Schmierstoffen und eine anschließende aufwändige Reinigung zum Zweck des Entfernens der Schmierstoffe bzw. zum Entfernen von Schneidspänen, so dass zusätzliche Arbeitsschritte anfallen. Gerade bei miniaturisierten Implantatkörpern ist dies oftmals mit einem erheblichen Aufwand, Schwierigkeiten und Kosten verbunden.

Es hat sich darüber hinaus gezeigt, dass vorbekannte Implantatkörper mit Wabenstruktur gemäß der eingangs geschilderten Art zu einer Subsidence, das heißt zu einem Einsinken des Implantates in die Wirbelkörper aufgrund zu hoher Flächenpressung führen.

Aus dem Vorgenannten ist es daher Aufgabe der Erfindung, einen weiterentwickelten, monolithischen zervikalen oder lumbalen Implantatkörper zur Fusion von Wirbelsäulensegmenten anzugeben, welcher unter Nutzung der an sich vorteilhaften Wabenstruktur ein unerwünschtes Einsinken in benachbarte Wirbelkörper oder Wirbelkörpersegmente vermeidet, dennoch sicher fixiert ist und darüber hinaus konstruktiv so umgesetzt wird, dass eine Fertigung mit rein additiven Verfahren ohne jegliche Nacharbeit möglich ist.

Die Lösung der Aufgabe der Erfindung erfolgt durch einen Implantatkörper gemäß der Merkmalskombination nach Patentanspruch 1, wobei die Unteransprüche mindestens zweckmäßige Ausgestaltungen und Weiterbildungen darstellen.

Es wird demnach von einem monolithischen, zervikalen oder lumbalen Implantatkörper ausgegangen, welcher zur Fusion von Wirbelsäulensegmenten dient.

Dieser Implantatkörper weist unter Einhaltung eines Zwischenraumes entsprechend beabstandete Ober- und Unterseiten auf.

Die Oberseite und die Unterseite sind durch Stege verbunden. Die Ober- und die Unterseite sind mit Ausnahme von formgebenden und strukturverstärkenden Randbereichen als Fläche, z.B. Wabenfläche, mit Durchbrüchen ausgebildet und können einen keilbildenden Winkel einschließen.

Mindestens eine Seitenfläche ist vollständig oder teilweise offen und dient dem Einbringen von Knochenersatzmaterial bzw. Knochenspänen.

Eine weitere Seitenfläche weist einen Stabilisierungsbereich auf. Dieser Stabilisierungsbereich stellt sich als entsprechend verstärkter Steg dar, der die Ober- und Unterseite des Implantatkörpers verbindet. Die Seitenfläche kann am Übergang zur Unterseite angeschrägt sein.

Im Stabilisierungsbereich ist eine Ausnehmung vorhanden. Diese Ausnehmung dient der Aufnahme eines chirurgischen Instrumentes während des Setzens des Implantates.

Es sind von den Oberflächen der formgebenden und strukturverstärkenden Randbereiche mehrere stiftförmige oder dornenförmige Fortsätze mit abgerundeten Spitzen in Richtung benachbarter Wirbelsäulenimplantate ausgebildet. Mit anderen Worten erstrecken sich diese Fortsetze im Wesentlichen senkrecht von der entsprechenden Oberfläche des strukturverstärkenden Randbereiches.

Die Ausnehmung zur Aufnahme eines chirurgischen Instrumentes ist erfindungsgemäß als durchgehendes oder unterbrochenes Rundgewinde ausgebildet. Hier handelt es sich bevorzugt um ein Rundgewinde aus zwei Radien, die tangential ineinander übergehen. Hierdurch werden scharfe Kanten, Ecken oder Überhänge durch die Flankenform sowohl am Implantat als auch am zugehörigen Instrument vermieden.

Aufgrund der Tatsache, dass es bei einem Rundgewinde keine filigranen Flanken gibt und dieses eine große Steigerung aufweist, ist ein solches Gewinde sehr robust, was beim Setzen des entsprechenden Implantates mit Handhabung der Instrumente einen maßgeblichen Vorteil darstellt.

Darüber hinaus beträgt die Wabenwandstärke der Wabenstruktur nur einen Bruchteil der Wabengröße. Die Wabengröße ist hier definiert als der Abstand zwischen gegenüberliegenden Wabenwänden einer betrachteten Wabe.

Bevorzugt liegt hier ein Verhältnis zwischen Wabenwandstärke zu Wabengröße von 1:10 vor.

Die Wabenwandstärke kann gezielt den Belastungen, die auf das Implantat einwirken, unterschiedlich stark gestaltet werden. Die Wabenrichtung kann senkrecht oder in einem abweichenden Winkel realisiert werden. Ebenso können gezielt einige Wabenwandungen weggelassen werden, um einen partiellen Durchgang zu schaffen, welcher der Fläche zweier oder mehrerer Waben entspricht.

In einer Weiterbildung der Erfindung weist die Oberseite eine ballige, im Wesentlichen konvexe Oberflächenform auf. Die Unterseite hingegen ist als Schrägfläche ausgebildet.

Der Schrägflächenwinkel liegt hier bei einem Bereich von im Wesentlichen 0 bis 15 Grad.

In einer möglichen Ausgestaltung des Implantatkörpers ist das Flächenmaß der Oberseite kleiner als das der Unterseite.

Der Stabilisierungsbereich mit Ausnehmung und Rundgewinde befindet sich im Wesentlichen mittig in der weiteren Seitenfläche.

In mindestens einem der die Ober- und Unterseite verbindenden Stege ist eine Ausnehmung oder ein Rücksprung zur Instrumentenführung ausgebildet. Eine weitere, zusätzliche Ausnehmung ist außermittig und unsymmetrisch angeordnet und gewährleistet zusammen mit einem entsprechend gestalteten Instrument eine lagegerechte Fixierung des Implantates auf dem Instrument.

Die erwähnten Fortsätze, die bevorzugt stiftförmig oder dornenförmig ausgebildet sind, befinden sich in den Eckbereichen des Implantatkörpers.

Die Fortsätze weisen an ihren freien Enden eine Abrundung auf. Mithin entsteht keine Keilspitze, sondern eher die Form eines Dorns bzw. Kegelstumpfes mit abgerundetem oberen Kegelende. Durch diese Ausbildung der Fortsätze ist das Implantat im entsprechenden benachbarten Wirbelkörper sicher gehalten, ohne dass eine punktuelle Keilwirkung entsteht, die womöglich den entsprechenden Wirbel nachhaltig schädigt.

Mindestens eine der Seitenflächen, insbesondere diejenige, welche der Seitenfläche mit Ausnehmung gegenüberliegt, ist geschlossen ausgeführt und bildet eine Produktkennzeichnungsfläche. Diese Kennzeichnungsfläche dient der Aufnahme von Angaben unter anderen zum Hersteller, zum LOT und weiteren Angaben.

Erfindungsgemäß wird der monolithische zervikale Implantatkörper einschließlich dem Rundgewinde frei von irgendwelchen nachgelagerten spangebenden Verfahren oder Verfahrensschritten allein durch selektives Laserschmelzen additiv gefertigt. Nacharbeiten, insbesondere auch im Gewindebereich, sind nicht erforderlich.

Die Erfindung soll nachstehend anhand von Ausführungsbeispielen sowie unter Zuhilfenahme von Figuren näher erläutert werden.

Hierbei zeigen:
- Fig. 1: eine Draufsicht auf die Oberseite eines beispielhaften Implantatkörpers;
- Fig. 2: eine Ansicht der weiteren Seitenfläche mit Stabilisierungsbereich und Gewindeausnehmung;
- Fig. 3: eine Schnittdarstellung längs der Linien A-A gemäß Fig. 2 mit erkennbarem Rundgewinde;
- Fig. 4: eine Detaildarstellung des Gewindes gemäß Fig. 3 (Detail A);
- Fig. 5: eine Seitenansicht auf den Implantatkörper mit offener Fläche zum Einbringen von Knochenersatzmaterial nebst erkennbaren Fortsätzen an der Ober- und der Unterseite sowie der balligen, konvexen Oberflächenform der Oberseite und der Ausbildung der Unterseite als Keil- oder Schrägfläche;
- Fig. 6: eine Darstellung der geschlossenen Implantat-Rückseitenfläche mit dort vorgesehener Möglichkeit zur Produktkennzeichnung;
- Fig. 7: eine Schnittdarstellung längs der Linie B-B gemäß Fig. 2 ebenfalls mit erkennbarem Rundgewinde und Fortsätzen;
- Fig. 8 und 9: perspektivische Darstellungen des Implantatkörpers in verschiedenen Ansichten;
- Fig. 10: beispielhafte Darstellungen verschiedener Schrägflächenwinkel im Bereich von 4,2 bis 12 Grad;
- Fig. 11 und 12: perspektivische Darstellungen in verschiedenen Ansichten einer weiteren Ausführungsform des erfindungsgemäßen Implantatkörpers mit geringerer höhenmäßiger Ausdehnung im Vergleich zum Ausführungsbeispiel gemäß den Fig. 1 bis 9;
- Fig. 13: Beispiele für Rundgewindeausführungen 1 bis 5; und
- Fig. 14a, b: Darstellungen eines beispielhaften unterbrochenen Rundgewindes.

Soweit in den figürlichen Darstellungen Abmessungen angegeben sind, sind diese lediglich beispielhaft und nicht den Erfindungsgedanken begrenzend oder einschränkend zu verstehen. Gleiches gilt für Angaben zum Maßstab in den figürlichen Darstellungen.

Der monolithische Implantatkörper 1 weist eine Oberseite 2 und eine Unterseite 3 auf.

Die Ober- und die Unterseite sind durch Stege 31 verbunden.

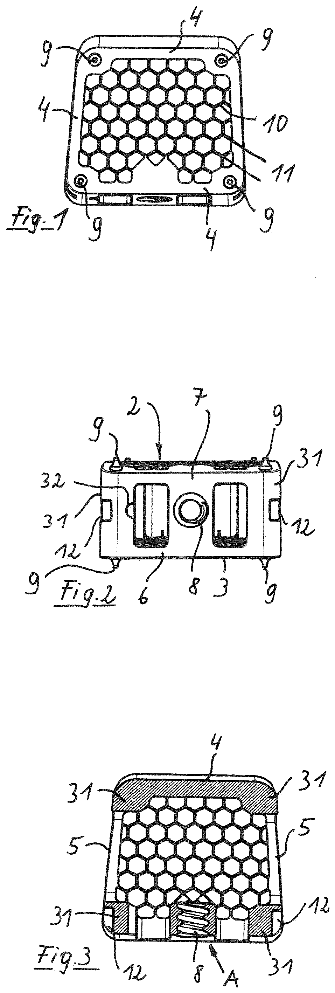

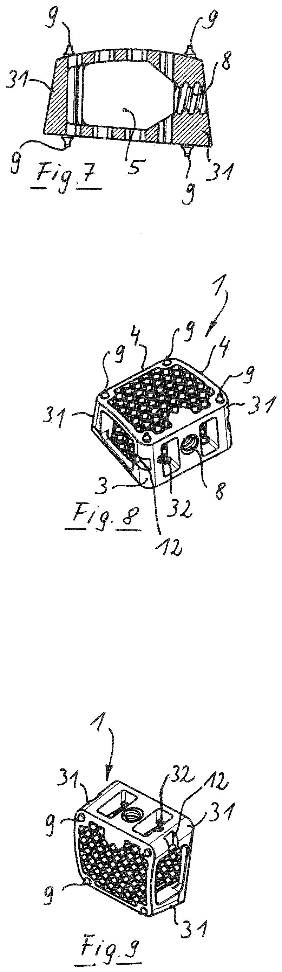

Mit Ausnahme von Randbereichen 4, die formgebend und strukturverstärkend sind, sind die Oberseite 2 und die Unterseite 3 als Wabenfläche ausgebildet, wie dies insbesondere den Figuren 1, 3, 8, 9 sowie 11 und 12 entnommen werden kann.

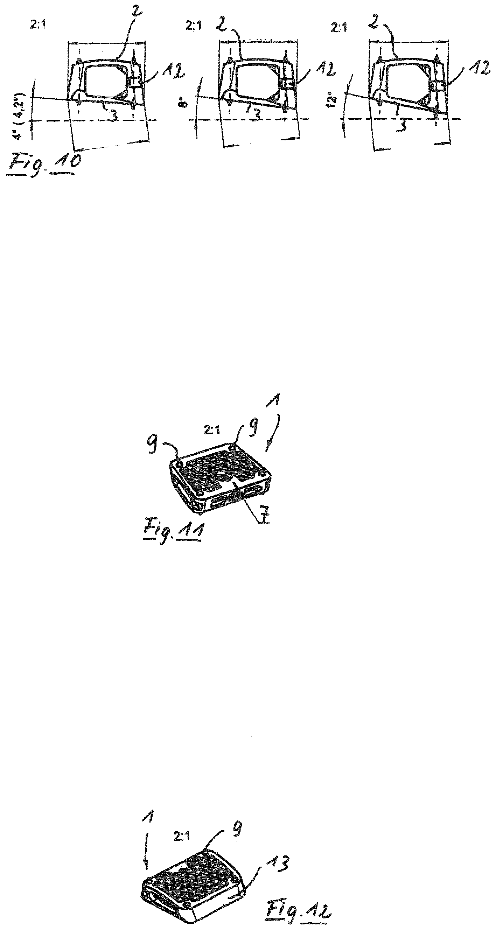

Die Oberseite 2 weist beispielsweise eine ballige Form 2 und die Unterseite 3 eine Schrägfläche auf, wobei die unterschiedlichen Schrägflächenwinkel, wie in der Figur 10 gezeigt, je nach anatomischen Gegebenheiten gewählt werden können. Beispielhaft sind Winkel von 4,2 bis 12 Grad möglich.

Gegenüberliegende Seitenflächen 5 sind weitgehend offen ausgebildet, so dass Ersatzmaterial oder Knochenzement in den inneren Freiraum des Implantatkörpers eingebracht werden kann.

Eine weitere Seitenfläche 6 besitzt einen Stabilisierungsbereich 7.

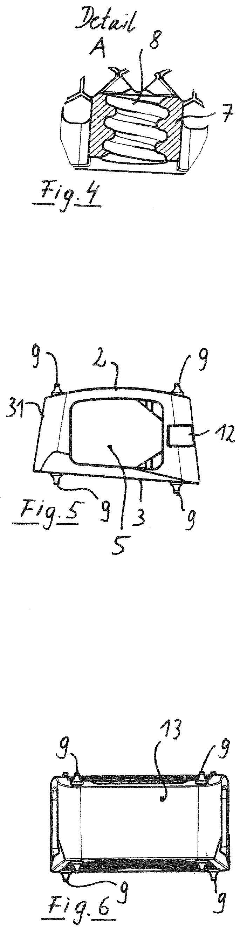

Im Stabilisierungsbereich 7 ist eine Ausnehmung, ausgebildet als Rundgewinde 8, vorhanden. Siehe hierzu die Darstellung nach Figur 3 mit dem Detail A nach Figur 4.

Ausgehend von den Oberflächen der formgebenden und strukturverstärkenden Randbereiche 4 sind mehrere stiftförmige Fortsätze 9 in Richtung benachbarter Wirbelsäulensegmente orientiert vorhanden.

Die Wabenwandstärke 10 beträgt nur einen Bruchteil der Wabengröße 11, die definiert ist als der Abstand zwischen gegenüberliegenden Wabenwänden.

Bevorzugt beträgt beispielsweise die Wabenstärke 0,15 mm und die Wabengröße 1,5 mm, so dass sich ein beispielhaftes Verhältnis von 1:10 ergibt.

Aus den Figuren ist ersichtlich, dass das Flächenmaß der Oberseite 2 größer als das Flächenmaß der Unterseite 3 sein kann.

Der Stabilisierungsbereich 7 mit Ausnehmung und Rundgewinde 8 befindet sich im Wesentlichen mittig in der weiteren Seitenfläche 6 wie dies aus den Figuren 1 bis 3 sowie 8 und 9, aber auch 11 nachvollzogen werden kann.

In einem der Stege 31, der die Oberseite 2 mit der Unterseite 3 verbindet, ist eine Ausnehmung oder ein Rücksprung 32 zur Instrumentenführung ausgebildet.

Die Fortsätze 9 befinden sich bevorzugt in den Eckbereichen des jeweiligen Implantatkörpers 1.

Mindestens eine der Seitenflächen ist geschlossen ausgeführt und bildet eine Produktkennzeichnungsfläche 13. Die geschlossene Seitenfläche, das heißt die Produktkennzeichnungsfläche 13, ist bevorzugt dem Stabilisierungsbereich 7 mit Ausnehmung und Rundgewinde 8 gegenüberliegend realisiert.

Der in den Ausführungsbeispielen gezeigte Implantatkörper wird einschließlich dem Rundgewinde 8 mittels selektiven Laserschmelzen additiv gefertigt. Spangebende oder andere, formverändernde Verfahren zur Bearbeitung oder Nachbearbeitung, insbesondere des Rundgewindes, sind nicht erforderlich.

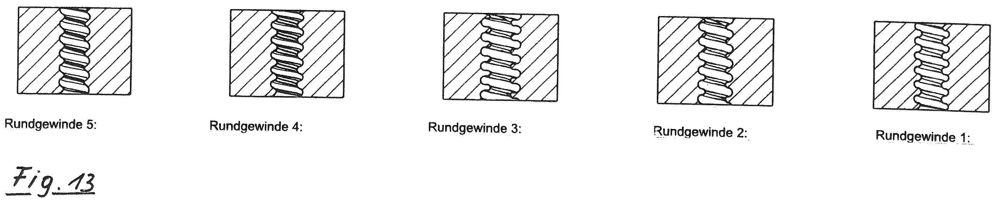

Mit Hilfe der Figur 13, die jeweils Schnittdarstellungen zeigt, soll ein Beispiel für Rundgewindeausführungen 1-5 erläutert werden.

Bei der Schnittdarstellung mit Rundgewinde 1 handelt es sich um einen Gewindegrundradius der gleich dem Radius an der Flankenspitze ist. Es ist ein unmittelbarer Übergang zwischen Gewindegrundradius und dem Radius der Flankenspitze gegeben.

Beim Rundgewinde 2 ist der Gewindegrundradius größer dem Radius an der Flankenspitze. Es ist eine dünne Flanke vorliegend. Weiterhin liegt ein unmittelbarer Übergang zwischen Gewindegrundradius und Radius der Flankenspitze vor.

Beim Rundgewinde 3 ist der Gewindegrundradius größer als der Radius an der Flankenspitze. Es liegt eine dünne Flanke vor. Weiterhin ist ein senkrechter Bereich zwischen Gewindegrundradius und Radius der Flankenspitze gegeben. Der Flankenwinkel beträgt hier annähernd 0 Grad. Die Flanke liegt senkrecht zur Gewindelängsachse.

Beim Rundgewinde 4 ist der Gewindegrundradius gleich dem Radius an der Flankenspitze. Der Flankenwinkel ist hier abweichend von 0 Grad.

Beim Rundgewinde 5 ist der Gewindegrundradius gleich dem Radius an der Flankenspitze. Der Flankenwinkel weicht hier von 0 Grad ab.

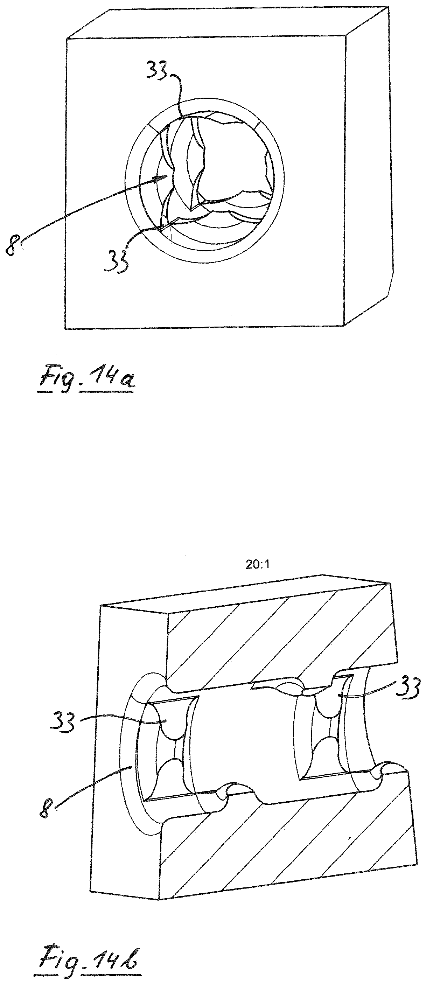

Die Figur 14a zeigt eine perspektivische Ansicht auf eine Ausnehmung mit Rundgewinde, wobei das Rundgewinde Unterbrechungen 33 aufweist. Mithin handelt es sich hier um ein unterbrochenes bzw. segmentiertes Rundgewinde zum leichteren Einführen des zugehörigen Instrumentes.

Die Figur 14b stellt einen Längsschnitt der Ansicht gemäß Figur 14a mit erkennbaren Unterbrechungen 33 dar.

## Patentansprüche

1. Monolithischer zervikaler oder lumbaler Implantatkörper (1) zur Fusion von Wirbelsäulensegmenten mit einer, voneinander unter Einhaltung eines Zwischenraumes beabstandeten Ober- (2) und Unterseite (3), wobei Ober-(2) und Unterseite (3) durch Stege (31) verbunden sind, weiterhin die Ober- (2) und Unterseite (3) mit Ausnahme von formgebenden und strukturverstärkenden Randbereichen (4) als Fläche mit Durchbrüchen ausgebildet sind und einen Winkel einschließen, mindestens einer vollständig oder teilweise offenen Seitenfläche (5) zum Einbringen von Knochenersatzmaterial und einer weiteren Seitenfläche (6) mit Stabilisierungsbereich (7) und einer Ausnehmung zur zeitweisen Aufnahme eines chirurgischen Instrumentes im Stabilisierungsbereich,
**dadurch gekennzeichnet, dass**
die Ausnehmung als durchgehendes oder unterbrochenes Rundgewinde (8) ausgebildet ist.

2. Monolithischer zervikaler oder lumbaler Implantatkörper nach Anspruch 1,
**dadurch gekennzeichnet, dass**
die Fläche als Wabenfläche ausgebildet ist und die Wabenwandstärke (10) einen Bruchteil der Wabengröße (11), definiert als der Abstand zwischen gegenüberliegenden Wabenwänden, bevorzugt in einem Verhältnis von im Wesentlichen 1:10 zwischen Wabenwandstärke (10) und Wabengröße (11) beträgt.

3. Monolithischer zervikaler oder lumbaler Implantatkörper nach Anspruch 1 oder 2,
**dadurch gekennzeichnet, dass**
ausgehend von den Oberflächen der formgebenden und strukturverstärkenden Randbereiche (4) sich mehrere stiftförmige oder dornenförmige Fortsätze (9) in Richtung benachbarter Wirbelsäulensegmente erstrecken.

4. Monolithischer zervikaler oder lumbaler Implantatkörper nach einem der vorangegangenen Ansprüche,
**dadurch gekennzeichnet, dass**
die Oberseite (2) eine ballige, konvexe Oberflächenform aufweist und die Unterseite (3) als Schrägfläche ausgebildet ist.

5. Monolithischer zervikaler oder lumbaler Implantatkörper nach Anspruch 4,
**dadurch gekennzeichnet, dass**
der Schrägflächenwinkel im Bereich zwischen im Wesentlichen 1 und 15 Grad, bevorzugt zwischen 4 und 12 Grad liegt.

6. Monolithischer zervikaler oder lumbaler Implantatkörper nach einem der vorangegangenen Ansprüche,
**dadurch gekennzeichnet, dass**
das Flächenmaß der Oberseite (2) kleiner als das Flächenmaß der Unterseite (3) ist.

7. Monolithischer zervikaler oder lumbaler Implantatkörper nach einem der vorangegangenen Ansprüche,
**dadurch gekennzeichnet, dass**
der Stabilisierungsbereich (7) mit Ausnehmung und Rundgewinde (8) sich im Wesentlichen mittig in der weiteren Seitenfläche (6) befindet.

8. Monolithischer zervikaler oder lumbaler Implantatkörper nach einem der Ansprüche 3 bis 7,
**dadurch gekennzeichnet, dass**
in mindestens einem der die Ober- und Unterseite (2; 3) verbindenden Stege (31) eine Ausnehmung oder einen Rücksprung (12) zur Instrumentenführung ausgebildet ist.

9. Monolithischer zervikaler oder lumbaler Implantatkörper nach einem der Ansprüche 3 bis 8,
**dadurch gekennzeichnet, dass**
die Fortsätze (9) jeweils in den Eckbereichen des Implantatkörpers (1) befindlich sind.

10. Monolithischer zervikaler oder lumbaler Implantatkörper nach einem der vorangegangenen Ansprüche,
**dadurch gekennzeichnet, dass**
mindestens eine Seitenfläche geschlossen ausgeführt ist und eine Produktkennzeichnungsfläche (13) bildet.

11. Monolithischer zervikaler oder lumbaler Implantatkörper nach Anspruch 8,
**dadurch gekennzeichnet, dass**
die geschlossene Seitenfläche dem Stabilisierungsbereich (7) mit Ausnehmung und Rundgewinde (8) gegenüberliegt.

12. Monolithischer zervikaler oder lumbaler Implantatkörper nach einem oder mehreren der vorangegangenen Ansprüche,
**dadurch gekennzeichnet, dass**
dieser einschließlich dem Rundgewinde (8) frei von spangebenden Verfahren durch selektives Laserschmelzen additiv gefertigt ist.

## Claims

1. A monolithic cervical or lumbar implant body (1) for fusing spinal column segments, comprising an upper side (2) and a lower side (3) which are spaced apart from one another while maintaining an intermediate space, wherein the upper side (2) and the lower side (3) are connected by webs (31), furthermore the upper side (2) and the lower side (3), with the exception of shape-defining and structure-reinforcing edge regions (4), are designed as a surface having openings and enclose an angle, at least one completely or partially open side surface (5) for introducing bone substitute material, and a further side surface (6) having a stabilisation region (7) and a recess for temporarily receiving a surgical instrument in the stabilisation region,
**characterized in that**
the recess is formed as a continuous or interrupted round thread (8).

2. The monolithic cervical or lumbar implant body according to Claim 1,
**characterized in that**
the surface is configured as a honeycomb surface, and the honeycomb wall thickness (10) is a fraction of the honeycomb size (11), defined as the distance between opposite honeycomb walls, preferably in a ratio of substantially 1:10 between the honeycomb wall thickness (10) and the honeycomb size (11).

3. The monolithic cervical or lumbar implant body according to Claim 1 or 2,
**characterized in that**
starting from the surfaces of the shape-defining and structure-reinforcing edge regions (4), several pin-shaped or mandrel-shaped protrusions (9) extend in the direction of adjacent spinal column segments.

4. The monolithic cervical or lumbar implant body according to one of the preceding claims,
**characterized in that**
the upper side (2) has a spherical, convex surface shape, and the lower side (3) is configured as an inclined surface.

5. The monolithic cervical or lumbar implant body according to Claim 4,
**characterized in that**
the inclined surface angle lies in the range between substantially 1 and 15 degrees, preferably between 4 and 12 degrees.

6. The monolithic cervical or lumbar implant body according to one of the preceding claims,
**characterized in that**
the surface dimension of the upper side (2) is smaller than the surface dimension of the lower side (3).

7. The monolithic cervical or lumbar implant body according to one of the preceding claims,
**characterized in that**
the stabilisation region (7) having the recess and the round thread (8) is located substantially centrally in the further side surface (6).

8. The monolithic cervical or lumbar implant body according to one of Claims 3 to 7,
**characterized in that**
in at least one of the webs (31) connecting the upper side and the lower side (2; 3), a recess or a setback (12) is formed for guiding an instrument.

9. The monolithic cervical or lumbar implant body according to one of Claims 3 to 8,
**characterized in that**
the protrusions (9) are respectively located in the corner regions of the implant body (1).

10. The monolithic cervical or lumbar implant body according to one of the preceding claims,
**characterized in that**
at least one side surface is embodied in a closed manner and forms a product labelling surface (13).

11. The monolithic cervical or lumbar implant body according to Claim 8,
**characterized in that**
the closed side surface lies opposite the stabilisation region (7) having the recess and the round thread (8).

12. The monolithic cervical or lumbar implant body according to one or more of the preceding claims,
**characterized in that**
it is additively manufactured including the round thread (8) free of machining methods by selective laser melting.

## Revendications

1. Corps d'implant (1) cervical ou lombaire d'un seul tenant pour la fusion de segments vertébraux comprenant une face supérieure (2) et une face inférieure (3) à distance l'une de l'autre par le maintien d'un espace intermédiaire, dans lequel la face supérieure (2) et la face inférieure (3) sont reliées par des montants (31), en outre la face supérieure (2) et la face inférieure (3) sont formées en tant que surface avec des percées, à l'exception de zones de bord (4) donnant la forme et renforçant la structure et incluent un angle, au moins un côté complètement ou partiellement ouvert (5) pour introduire un matériau de substitut osseux et un côté (6) supplémentaire avec une zone de stabilisation (7) et un évidement pour la réception temporaire d'un instrument chirurgical dans la zone de stabilisation,
**caractérisé en ce que**
l'évidement est conçu en tant que filetage rond (8) continu ou interrompu.

2. Corps d'implant cervical ou lombaire d'un seul tenant selon la revendication 1,
**caractérisé en ce que**
la surface est conçue en tant que surface alvéolaire et l'épaisseur de paroi alvéolaire (10) fait une fraction de la taille d'alvéole (11), définie en tant que la distance entre des parois alvéolaires opposées, de préférence dans un rapport d'essentiellement 1:10 entre l'épaisseur de paroi alvéolaire (10) et la taille d'alvéole (11).

3. Corps d'implant cervical ou lombaire d'un seul tenant selon la revendication 1 ou 2,
**caractérisé en ce**
**qu'**en partant des surfaces des zones de bord (4) donnant la forme et renforçant la structure, plusieurs prolongements (9) en forme de tige ou d'épine s'étendent en direction de segments vertébraux voisins.

4. Corps d'implant cervical ou lombaire d'un seul tenant selon l'une des revendications précédentes,
**caractérisé en ce que**
la face supérieure (2) présente une forme de surface convexe bombée, et la face inférieure (3) est conçue en tant que surface biseautée.

5. Corps d'implant cervical ou lombaire d'un seul tenant selon la revendication 4,
**caractérisé en ce que**
l'angle de surface biseautée est situé dans la plage entre essentiellement 1 et 15 degrés, de préférence entre 4 et 12 degrés.

6. Corps d'implant cervical ou lombaire d'un seul tenant selon l'une des revendications précédentes,
**caractérisé en ce que**
l'unité d'aire de la face supérieure (2) est inférieure à l'unité d'aire de la face inférieure (3).

7. Corps d'implant cervical ou lombaire d'un seul tenant selon l'une des revendications précédentes,
**caractérisé en ce que**
la zone de stabilisation (7) avec l'évidement et le filetage rond (8) se trouve essentiellement au centre dans le côté (6) supplémentaire.

8. Corps d'implant cervical ou lombaire d'un seul tenant selon l'une des revendications 3 à 7,
**caractérisé en ce que**
dans au moins un des montants (31) reliant les faces supérieure et inférieure (2 ; 3), un évidement ou une avancée (12) est formée pour guider des instruments.

9. Corps d'implant cervical ou lombaire d'un seul tenant selon l'une des revendications 3 à 8,
**caractérisé en ce que**
les avancées (9) se trouvent respectivement dans les zones d'angle du corps d'implant (1).

10. Corps d'implant cervical ou lombaire d'un seul tenant selon l'une des revendications précédentes,
**caractérisé en ce**
**qu'**au moins un côté est conçu fermé et forme une face d'identification de produit (13).

11. Corps d'implant cervical ou lombaire d'un seul tenant selon la revendication 8,
**caractérisé en ce que**
le côté fermé est opposé à la zone de stabilisation (7) avec l'évidement et le filetage rond (8).

12. Corps d'implant cervical ou lombaire d'un seul tenant selon l'une ou plusieurs des revendications précédentes,
**caractérisé en ce que**
celui-ci, y compris le filetage rond (8) est fabriqué de manière additive par fusion au laser sélective dans un procédé sans copeaux.
